# EUROPÄISCHE PATENTSCHRIFT

(11) **EP 1 148 911 B1**
(45) Veröffentlichungstag und Bekanntmachung des Hinweises auf die Patenterteilung: **22.12.2004**
(21) Anmeldenummer: 00905015.4
(22) Anmeldetag: 03.02.2000
(51) Int. Cl.: A61N 5/10

(54) **GANTRY-SYSTEM**
GANTRY SYSTEM
SYSTEME DE PORTIQUE

(30) Priorität: 04.02.1999 DE 19904675
(43) Veröffentlichungstag der Anmeldung: 31.10.2001
(73) Patentinhaber: GSI Gesellschaft für Schwerionenforschung mbH, 64291 Darmstadt-Wixhausen (DE)
(72) Erfinder: PAVLOVIC, Marius, D-64291 Darmstadt (DE); SCHARDT, Dieter, D-64291 Darmstadt (DE)
(74) Vertreter: Boeters, Hans Dietrich, Dr.
(86) Internationale Anmeldenummer: PCT/EP2000/000866
(87) Internationale Veröffentlichungsnummer: WO 2000/045891

(56) Entgegenhaltungen:
- EP-A- 0 635 849
- EP-A- 0 864 337
- US-A- 4 870 287
- PAVLOVIC M: "Oblique gantry - an alternative solution for a beam delivery system for heavy-ion cancer therapy" NUCLEAR INSTRUMENTS & METHODS IN PHYSICS RESEARCH, SECTION - A: ACCELERATORS, SPECTROMETERS, DETECTORS AND ASSOCIATED EQUIPMENT,NL,NORTH-HOLLAND PUBLISHING COMPANY. AMSTERDAM, Bd. 434, Nr. 2-3, 21. September 1999 (1999-09-21), Seiten 454-466, XP004180276 ISSN: 0168-9002

## Beschreibung

Die Erfindung betrifft ein Gantry-System zum Einstellen und Ausrichten eines Ionenstrahls auf ein Target gemäß dem Oberbegriff des Anspruchs 1.

Ein derartiges Gantry-System ist aus der US 4,870,287 bekannt. Bei dem bekannten Gantry-System wird der Ionenstrahl in der horizontal angeordneten Gantry-Rotationsachse dem Gantry-System zugeführt und mittels Magnetoptiken von der Gantry-Rotationsachse zunächst abgelenkt.

Danach wird der Ionenstrahl mittels Magnetoptiken parallel zur Gantry-Rotationsachse geführt und schließlich aus dieser parallelen Richtung zur Gantry-Rotationsachse in eine radiale Richtung zur Gantry-Rotationsachse abgelenkt. Das Target wird üblicherweise in dem Schnittpunkt des radial geführten Ionenstrahls mit der Gantry-Rotationsachse angeordnet. Dieser Schnittpunkt wird als Isozentrum definiert.

Der Ionenstrahl kann somit bei einer vollständigen Umdrehung der Gantry um die Gantry-Rotationsachse auf das Target in einer Ebene senkrecht zur Gantry-Rotationsachse ausgerichtet werden und auf Winkel zwischen 0 und 360° eingestellt werden.

Das Gantry-System weist außer der Gantry ein Targetträger-System mit einem drehbaren Targetträger auf. Die Träger-Rotationsachse des Targetträgers ist vertikal zu der Gantry-Rotationsachse in dem Isozentrum angeordnet. Somit kann das Gantry-System, das mindestens eine Gantry und ein Targetträger-System aufweist, einen Ionenstrahl derart einstellen und ausrichten, daß ein im Isozentrum angeordnetes Target aus einem beliebig bestimmbaren Winkel im Raum bestrahlt werden kann. Bei einem derartigen Gantry-System ist es notwendig, daß der letzte Ablenkungsmagnet der Gantry den Ionenstrahl um 90° ablenkt, weshalb eine derartige Gantry auch 90°-Gantry genannt wird.

Bei der aus der Druckschrift US 4,870,287 bekannten 90°-Gantry verläßt der Ionenstrahl folglich die Gantry in Richtung auf die Gantry-Rotationsachse senkrecht zu der Gantry-Rotationsachse. Ein Winkel α der Gantryrotation wird zwischen der Ebene, in der der Ionenstrahl durch die Gantry geführt wird, und der horizontalen Ebene des Raums, in dem sich die Gantry-Rotationsachse befindet, definiert. Demnach entspricht eine horizontale Position der Gantry entweder dem Winkel α = 0 oder dem Winkel α = 180°, wenn die Gantry in der horizontalen Ebene liegt und damit der Ionenstrahl in der Gantry in dieser horizontalen Ebene geführt wird. Demgemäß entspricht die oberste Position der Gantry in vertikaler Richtung dem Winkel α = 90° und die unterste Position der Gantry weist einen Winkel von α = 270° auf.

Ein Behandlungswinkel γ ist zwischen der horizontalen Ebene des Raumes und der Richtung, in welcher der Ionenstrahl in ein Targetvolumen eintritt, definiert. Ein effektiver Behandlungswinkel ist zwischen einer frontalen Ebene eines Patienten und der Richtung, in welcher der Ionenstrahl in ein Targetvolumen eintritt, definiert. Für einen üblicherweise liegenden Patienten sind der Behandlungswinkel und der effektive Behandlungswinkel identisch.

Bei dem aus der Druckschtrift US 4,870,287 bekannten 90°-Gantry-System ist der Targetträger als um eine vertikale Achse drehbarer Tisch mit einer Längsachse und einer Querachse ausgebildet. Ein Winkel β der Targetträgerrotation ist zwischen der Längsachse des Targetträgertisches und der Gantry-Rotationsachse definiert. Aufgrund der Drehbarkeit des Targetträgers um eine vertikale Achse kann der winkel β Werte zwischen 0° und 360° einnehmen. Für einen vorgegebenen Behandlungswinkel γ, der von dem Gantry-Rotationswinkel α abhängt kann weiterhin ein bestimmter Eintrittskanal für die Tumorbestrahlung durch Einstellen des Winkels β der Trägerrotation gewählt werden. Aufgrund der Einstellbarkeit des Winkels β, der der Targetträgerrotation zugeordnet ist, und der Einstellbarkeit des Winkels α, der der Gantryrotation zugeordnet ist, kann bei einem konventionellen System, bei dem der Ionenstrahl durch den letzten Ablenkungsmagneten radial zur Gantry-Rotationsachse abgelenkt wird, das Targetvolumen, das auf dem Targetträger fixiert ist, für jeden Eintrittskanal zur Tumorbehandlung ausgerichtet werden.

Das aus der Druckschtrift US 4,870,287 bekannte 90°-Gantry-System hat den Nachteil, daß der letzte Ablenkungsmagnet der Gantry den Ionenstrahl um mindestens 90° ablenken muß, um alle Behandlungswinkel γ in einem Gantry-System mit Targetträger-System zu ermöglichen. Der große Ablenkungswinkel des letzten Ablenkungsmagneten erfordert abhängig von der Massenzahl der abzulenkenden Ionen einen großen Radius oder eine hohe magnetische Feldstärke. Damit ist der Nachteil verbunden, daß einerseits die Konstruktion einer Gantry bisher nur für Ionen mit kleinster Massenzahl gelungen ist, das heißt, für Protonen, und für Ionen mit höherer Massenzahl zwischen 4 und 16 der letzte Ablenkungsmagnet den Umfang und die Masse der Gantry wegen der schweren Ionen mit höherer Massenzahl als ein Proton derart aufbläht, daß ein Gantry-System für die klinische Anwendung nicht mehr vertretbar ist.

Zur Verringerung von Masse und Volumen einer Gantry für Ionen schwerer als Protonen gibt es Vorschläge, supraleitende Materialien für die Erregerspulen der Ablenkungsmagnete einzusetzen. Damit würden zwar die zu rotierenden Massen und das Volumen der Gantry verringert, jedoch würden die Kosten für die Kühlung der supraleitenden Materalien das Gantry-System in erheblichem Maße verteuern, zumal eine 360° Rotation für ein Kühlsystems mit flüssigem Helium oder flüssigem Stickstoff für moderne supraleitende Materialien äußerst problematisch ist.

Ein weiterer Vorschlag, der in der japanischen Veröffentlichung im Journal of the Japanese Society for Therapeutic Radiology and Oncology, vol. 9, suppl. 2, November 1997 im Rahmen der Proceedings of the XXVII PTCOG Meeting von M. Pavlovic unter dem Titel "GSI Studies of a Gantry for Heavy Ion Cancer Therapy" vorgetragen wurde, ermöglicht das Vermindern der Masse und des Volumens der Gantry durch Ändern des Ablenkungsgrades des letzten Ablenkungsmagneten von bisher 90° auf 60°. Diese Lösung hat den Nachteil, daß der Behandlungswinkel γ nur noch von 0° bis 60° mit einer derartigen sogenannten 60°-Gantry im Zusammenwirken mit dem konventionellen Targetträger-System realisiert werden kann. Somit sind mit einem derartigen Gantry-System, das einen Ablenkungswinkel von 60° für den letzten Ablenkungsmagneten aufweist, Behandlungswinkel γ zwischen größer 60° und 90° nicht mehr realisierbar

Aufgabe der Erfindung ist es, mit einer Gantry mit vermindertem Ablenkungswinkel des letzten Ablenkungsmagneten ein Gantry-System gemäß dem Oberbegriff des Anspruchs 1 anzugeben, das ohne supraleitende Materialien für die Magnetoptiken auskommt und trotz Verkleinern des Ablenkungswinkels des letzten Ablenkungsmagneten unter 90° ein Einstellen und Ausrichten eines Ionenstrahls auf ein Target aus einem beliebig bestimmbaren effektiven Behandlungswinkel ermöglicht. Ferner ist es Aufgabe der Erfindung, ein Verfahren zur Bestrahlung eines Targetvolumens und Einstellen und Ausrichten eines Ionenstrahls zur Behandlung eines Tumors mit Hilfe des erfindungsgemäßen Gantry-Systems anzugeben.

Diese Aufgabe wird mit den Merkmalen des Gegenstandes der Ansprüche 1 und 7 gelöst.

Dazu lenkt der letzte Ablenkungsmagnet den Ionenstrahl derart ab, daß er unter einem Winkel zwischen größer gleich 45° und kleiner 90° die Gantry-Rotationsachse im Isozentrum schneidet, so daß der Ionenstrahl beim Drehen der Gantry um eine volle Umdrehung um die Gantry-Rotationsachse einen Kegelmantel beschreibt und das Targetträger-System für zwei Positionen, die in vertikaler Ebene senkrecht zueinander sind, einen Targetträger aufweist, wobei dessen Träger-Rotationsachse in das Isozentrum des Gantry-Systems bringbar ist.

Eine derartige Lösung hat den Vorteil, daß der Targeträger nur in zwei festliegenden Positionen fixierbar sein muß und in beiden Positionen, die in vertikaler Ebene senkrecht zueinander sind, um eine vertikal ausgerichtete Träger-Rotationsachse drehbar sein muß. Ein wesentlicher Vorteil dieses Gantry-Systems ist es, daß selbst Winkel, die kleiner als 90° und vorzugsweise kleiner als 60°sind und somit vorteilhaft ein äußerst niedriges Gantryvolumen und äußerst geringe Abmessungen für den Durchmesser einer Gantry ermöglichen, mit dem erfindungsgemäßen Gantry-System realisiert werden können. Ein derartig kompaktes Gantry-System benötigt keine teuren Hilfsaggregate zur Kühlung von supraleitenden Materialien. Ein weiterer vorteil einer derartigen Gantry mit konventionellen Magnetoptiken ist, daß nun auch Ionenstrahlen von Ionen schwerer als Protonen mit Massenzahlen zwischen 4 und 16 durch ein Gantry-System, das für klinische Maßstäbe geeignet ist, für jeden beliebig bestimmbaren effektiven Behandlungswinkel einstellbar und ausrichtbar sind.

Um ein Targetvolumen mit einer optimalen Dosisverteilung zu bestrahlen, ist bei dem erfindungsgemäßen Gantry-System vorzugsweise eine Auslenkung des Ionenstrahls vorgesehen, um damit schichtweise das Targetvolumen abzutasten. Die Führung des Ionenstrahls in der Gantry vom Einkopplungspunkt des Ionenstrahls in die Gantry-Rotationsachse bis zur Umlenkung des Ionenstrahls im letzten Ablenkungsmagneten des Gantry-Systems erfolgt vorzugsweise dadurch, daß der Ionenstrahl zunächst mit einem 38°-Ablenkungsmagneten von der Gantry-Rotationsachse abgelenkt und mit einem zweiten 38°-Ablenkungsmagneten in eine Richtung parallel zur Gantry-Rotationsachse gebracht wird. In dieser parallelen Richtung durchläuft der Ionenstrahl zwei Scannermagnete, die den Ionenstrahl in zwei senkrecht aufeinanderstehenden Richtungen (horizontal und vertikal zum Ionenstrahl), die ihrerseits orthogonal zum Ionenstrahl verlaufen, ablenken, so daß ein Abtasten einer Fläche des Targetvolumens nach Durchlaufen des gescannten Ionenstrahls durch den letzten Ablenkungsmagneten in vorteilhafter Weise möglich wird.

Die bevorzugte Positionierung des Abtastsystems stromaufwärts des letzten Ablenkungsmagneten vermindert somit beträchtlich den Gantryradius und erfordert jedoch eine vergrößerte Apertur, um ein großes Behandlungsfeld zu ermöglichen. Diese bevorzugte Anordnung von Ablenkungsmagneten und Abtastsystemen zeigt einen hohen Grad von ionenoptischer Flexibilität. Der Ionenstrahl kann im Isozentrum deshalb vorteilhaft von 2 bis 16 mm Durchmesser angepaßt werden und die Magnetoptiken der Gantry sind immer achromatisch.

In einer bevorzugten Ausführungsform der Erfindung lenkt der letzte Ablenkungsmagnet den Ionenstrahl derart ab, daß die Gantry-Rotationsachse unter einem Winkel größer gleich 45° und kleiner 60° im Isozentrum geschnitten wird. Insbesondere die Ablenkungswinkel unter 60° zeigen die enormen Vorteile der vorliegenden Erfindung, indem einerseits die Gantryabmessungen minimiert werden und andererseits das Gantry-System in Zusammenwirkung der bevorzugten Ausführungsform der Gantry mit dem erfindungsgemäßen Targetträgersystem ein Einstellen und Ausrichten eines Ionenstrahls auf ein Target aus einem beliebig bestimmbaren effektiven Behandlungswinkel sicherstellt.

In einer bevorzugten Ausführungsform der Erfindung weist das Targetträgersystem eine Drehbühne auf, die um eine vertikale Drehbühnenachse drehbar ist. Auf dieser Drehbühne sind zwei Targetträger in zwei Positionen angeordnet, wobei die Positionen in vertikaler Ebene senkrecht zueinander sind. Jeder der beiden Targetträger ist um eine vertikale Träger-Rotationsachse drehbar.

Dieses bevorzugte Targetträger-System sieht vor, daß je nach erffektivem Behandlungswinkel ein Patient auf dem Targetträger entweder in liegender oder in sitzender Position angeordnet wird. Der effektive Behandlungswinkel muß aus medizinischen Gründen ein beliebig bestimmbarer Winkel im Patientenkoordinatensystem sein, um möglichst eine optimale Abtastung des Targetvolumens durch den Ionenstrahl zu gewährleisten.

Die Optimierung der Ionenstrahldosisverteilung in dem Targetvolumen oder einem Volumenelement des Targets hängt wesentlich von der Struktur des zu durchstrahlenden gesunden Gewebes über dem Tumorvolumen ab. Demnach sind bei der medizinischen Festlegung des effektiven Behandlungswinkels und des Eintrittskanals Gewebehohlräume und Gewebeverdichtungen, beispielsweise bei Knochengewebe, sowie die Lage kritischer Organe in der Nähe des Tumors entsprechend zu berücksichtigen. Insofern ist ein beliebig bestimmbarer effektiven Behandlungswinkel, der durch das erfindungsgemäße Gantry-System ermöglicht wird, ein großer Vorteil für eine klinische Behandlung.

vorzugsweise sind die vertikalen Träger-Rotationsachsen der zwei Positionen der Targetträger wechselweise in das Isozentrum des Gantrysystems mittels einer Drehbühne durch Drehen der Drehbühne um ihre Drehbühnenachse bringbar. Mit dieser bevorzugten Ausführungsform ist der Vorteil verbunden, daß der Patient in einer der beiden Positionen auf dem entsprechenden Targetträger positioniert werden kann und anschließend mittels der Drehbühne in das Isozentrum des Gantry-Systems auf dem Targetträger bringbar ist. Dann kann die Gantry auf den vorher bestimmten Winkel α eingestellt werden, und der Targetträger in der gewählten Position auf den vorbestimmten Winkel β mittels einer Drehbewegung um die vertikale Trägerrotationsachse eingestellt werden. Nach diesen drei Einstellungen kann dann das Targetvolumen mit dem gescannten Ionenstrahl abgetastet werden.

In einer weiteren bevorzugten Ausführungsform der Erfindung weist das Targetträgersystem zwei getrennte Zuführschienen auf, auf denen jeweils ein Targetträger der zwei Positionen angeordnet ist, wobei die Zuführschienen aus jeweils unterschiedlichen Richtungen jeweils einen der Targetträger mit seiner Rotationsachse in das Isozentrum wechselweise lateral verschieben können. Dieses System hat den Vorteil, daß der Patient auf dem ausgewählten Targetträger außerhalb des Isozentrums positioniert und präpariert werden kann und dann durch Verschieben auf den Zuführschienen in das Isozentrum gebracht werden kann. In dem Isozentrum kann dann das Abtasten des Targetvolumens vorgenommen werden, wenn zwischenzeitlich der Gantryrotationswinkel α mittels Drehen der Gantry und β mittels Drehen des Targetträgers eingestellt wurden.

In einer anderen bevorzugten Ausführungsform weist das Targetträgersystem ein universales Roboter-System auf, das den Targetträger in zwei Positionen und die vertikalen Träger-Rotationsachsen in dem Isozentrum anordnet. Derartige mehrachsige Robotersysteme ermöglichen es, einen drehbaren Targetträger in unterschiedlichen Positionen in dem Isozentrum anzuordnen und ersetzt damit die sonst notwendigen, unterschiedlich konstruierten zwei Targetträger und eventuelle Zuführschienen oder Drehbühnen. Ein derart universales Robotersystem kann für diese bevorzugte Anwendung stark vereinfacht werden, zumal nur zwei in der vertikalen Ebene senkrechtzueinander stehende Positionen des drehbaren Targetträgers erforderlich werden.

Das Verfahren zum Bestrahlen eines Tumors aus beliebig bestimmbarem effektivem Behandlungswinkel mittels eines Gantry-Systems ist durch folgende Verfahrensschritte gekennzeichnet:
a) Bestimmen des günstigsten effektiven Behandlungswinkels und des günstigsten Eintrittskanals in bezug auf die Lage und Größe eines Tumors in einem gesunden Gewebe und unter der Maßgabe einer minimalen Strahlenbelastung des umgebenden Gewebes mit einer optimalen Verteilung einer Ionenstrahldosis für das zu bestrahlende Tumorgewebe,
b) Auswahl der für den bestimmten effektiven Behandlungswinkel notwendigen Position des Targetträgers aus zwei in einer vertikalen Ebene senkrecht aufeinanderstehenden Positionen,
c) Verbringen der Trägerrotationsachse der geeigneten Position des Targetträgers in das Isozentrum des Gantry-Systems,
d) Ausrichten und Einstellen des Targetträgers durch Drehen des Targetträgers um seine vertikale Trägerrotationsachse in bezug auf den günstigsten Winkel,
e) Ausrichten und Einstellen der Gantry durch Drehen der Gantry um ihre horizontale Gantry-Rotationsachse in bezug auf den günstigsten Winkel,
f) Räumliches Abtasten des gesamten Tumorvolumens mit dem Ionenstrahl aus dem effektiven Behandlungswinkel.

Dieses Verfahren hat den Vorteil, daß mit Hilfe der Kombination eines auf Behandlungswinkel γ limitierten Gantry-Systems und eines Targetträger-Systems, das das Target in zwei in vertikaler Ebene senkrecht zueinanderliegenden Positionen dem Isozentrum der Gantry zuführt, jeder beliebig bestimmbare effektive Behandlungswinkel zur Bestrahlung des Tumorvolumens einstellbar ist, so daß ohne Einschränkungen des effektiven Behandlungswinkels ein Target mit Ionen bestrahlt werden kann. Dabei ist es unerheblich, ob zuerst der Winkel α mit Hilfe der Gantry und anschließend der Winkel β mit Hilfe des Targetträgers eingestellt wird, oder die umgekehrte Reihenfolge gewählt wird.

Wesentlich ist lediglich, daß für effektive Behandlungswinkel von 0 bis 90° minus λ nur eine Position und damit vorzugsweise einer der Targetträger einsetzbar ist und für Winkel zwischen 90° minus λ und dem Ablenkungswinkel λ des letzten Ablenkungsmagneten beide Positionen des Targetträgers anwendbar sind und für effektive Behandlungswinkel zwischen dem Ablenkungswinkel des letzten Ablenkungsmagneten und 90° die andere der beiden Positionen für den Targetträger verwendbar ist. Der wesentliche vorteil dieses Verfahrens ist folglich, daß trotz eingeschränktem Ablenkungswinkel des letzten Ablenkungsmagneten der zu behandelnde Tumor mit einem Ionenstrahl aus jeder beliebig bestimmbaren Richtung behandelt werden kann und somit die medizinisch optimale Bestrahlungsrichtung mit einem in seiner Massse, seinem Volumen und seinen Kosten verminderten Gantry-System eingehalten werden kann.

Weitere Vorteile, Merkmale und Anwendungsmöglichkeiten der Erfindung werden nun anhand von Ausführungsbeispielen unter Bezugnahme auf die beigefügten Zeichnungen näher erläutert.
Fig. 1 zeigt eine perspektivische Ansicht einer Ausführungsform des Gantry-Systems gemäß der Erfindung,
Fig. 2 zeigt einen Vergleich der Wirkung des letzten Ablenkungsmagneten einer herkömmlichen Gantry und einer Gantry, die bei dem erfindungsgemäßen Gantry-System zum Einsatz kommt,
Fig. 3 zeigt die Winkelverhältnisse und Winkeldefinitionen einer Gantry, wie sie beim erfindungsgemäßen Gantry-System zum Einsatz kommen,
Fig. 4 zeigt die Ionenstrahlführung der Gantry bei α = 90°,
Fig. 5 zeigt die Ionenstrahlführung der Gantry bei α = 45°,
Fig. 6 zeigt eine Seitenansicht einer der beiden Positionen des Targetträgers,
Fig. 7 zeigt eine Seitenansicht der anderen der beiden Positionen des Targetträgers,
Fig. 8 zeigt eine Anordnung von Zuführschienen gemäß einer weiteren Ausführungsform der Erfindung.

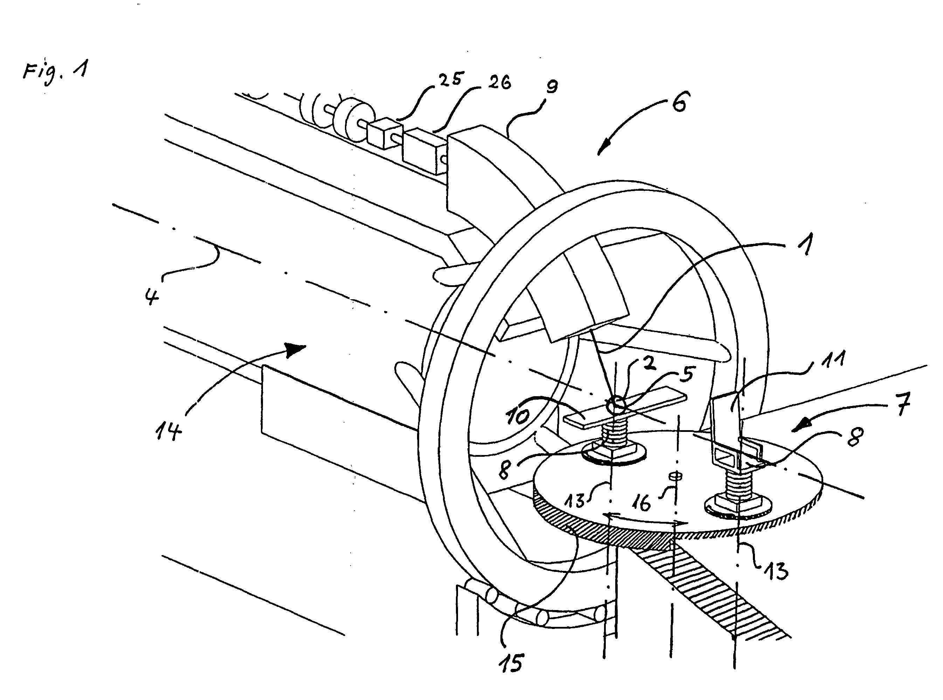

Fig. 1 zeigt eine perspektivische Ansicht einer Ausführungsform des Gantry-Systems 6 der Erfindung. Ein derartiges Gantry-System 6 richtet einen Ionenstrahl 1 auf ein Target 2. Dazu wird der Ionenstrahl 1 in der horizontal angeordneten Gantry-Rotationsachse 4 dem Gantry-System 6 zugeführt und mittels Magnetoptiken von der Gantry-Rotationsachse 4 zunächst abgelenkt und dann parallel zur Rotationsachse des Gantry-Systems geführt. Die Gantry 14 kann eine volle Umdrehung unter einem einstellbaren Winkel α von 0 bis 360° ausführen. Bei einer derartigen Umdrehung beschreibt der Ionenstrahl einen Kegelmantel, dessen Kegelspitze auf der Gantry-Rotationsachse liegt und diese im Isozentrum 5 schneidet. Das Gantry-System 6 weist neben der Gantry 14 ein Targetträger-System 7 auf. Dieses Target-Trägersystem 7 hat mindestens einen drehbaren Targetträger 8, dessen Rotationsachse 13 vertikal zu der Gantry-Rotationsachse 4 in dem Isozentrum angeordnet werden kann.

Der letzte Ablenkungsmagnet 9 lenkt den Ionenstrahl 1 derart ab, daß er unter einem Winkel zwischen größer gleich 45° und kleiner 90° die Gantry-Rotationsachse 4 im Isozentrum 5 schneidet. In der abgebildeten Ausführungsform ist dieser Ablenkungswinkel 55°, so daß der Ionenstrahl 1 beim Drehen des Gantry-Systems 6 um eine volle Umdrehung um die Rotationsachse 4 einen Kegelmantel eines Kegels mit einem Öffnungswinkel von 110° beschreibt. In der Fig. 1 beträgt die Einstellung des Winkels α 90°, so daß das Ionenstrahlablenksystem der Gantry 14 in seiner obersten Position angeordnet ist. Ein Target 2, das auf dem Targetträger 8 in der Position 10 angeordnet ist, kann um eine vertikale Achse 13 im Isozentrum gedreht werden und trotz vollständiger Umdrehungsmöglichkeit der Gantry von 0 bis 360° nur bis zu Behandlungswinkeln, die dem Austrittswinkel des Ionenstrahls aus den Magnetoptiken der Gantry entsprechen, bestrahlt werden. In der Ausführungsform mit einer 55°-Gantry ist dieser Austrittswinkel gegenüber einer horizontalen Ebene 55°, da der letzte Ablenkungsmagnet 9 den Ionenstrahl lediglich um diese 55° aus einer Paralelen zur Gantryrotationsachse ablenkt.

Mit einem Targetträger 8 in der Position 10 können deshalb nicht alle effektiven Behandlungswinkel erreicht werden. Deshalb weist diese bevorzugte Ausführungsform der Erfindung einen weiteren Targetträger 8 auf, der eine zweite Position 11 des Targets ermöglicht, die gegenüber der Position 10 in einer vertikalen Ebene senkrecht ausgerichtet ist. In dieser Position 11 kann das Target mit den effektiven Behandlungswinkeln beaufschlagt werden, die in der Position 10 nicht erreichbar sind. Das sind die effektiven Behandlungswinkel von 55° bis 90°. Darüber hinaus können die effektiven Behandlungswinkel von 35 bis 55° mit beiden Positionen 10 und 11 des Targetträgers 8 erreicht werden.

Somit ist es möglich, mit dieser Ausführungsform einen Ionenstrahl einzustellen und auf ein Target auszurichten, der aus einem beliebig bestimmbaren effektiven Behandlungswinkel trotz vermindertem Ablenkungswinkel des letzten Ablenkungsmagneten 9 auf das Target auftrifft.

Das gesamte Targetvolumen kann volumenelementweise und schictitweise durch eine scannbare magnetische horizontale Ablenkung 25, die stromaufwärts vom letzten Ablenkungsmagneten 9 angeordnet ist, und eine scannbare magnetische vertikale Ablenkung 26, die zwischen der horizontalen Ablenkung 25 und dem letzten Ablenkungsmagneten 9 angeordnet ist, abgetastet werden. Diese Abtastung erfolgt schichtweise, in dem die Eindringtiefe und damit die Schichthöhe im Target durch die jeweilige Energie des Ionenstrahls eingestellt wird.

Durch geeignete Programmierung der horizontalen 25 und der vertikalen Ablenkung 26 können die unterschiedlichsten Volumenformen des Targets abgetastet werden und damit messerscharf Tumorgewebe von umgebendem gesunden Gewebe unterschieden werden.

Aufgrund der erhöhten Massenträgheit von Ionen schwerer als Protonen hat ein Gantry-System mit einem Austrittswinkel nach dem letzten Ablenkungsmagneten von kleiner 90° den Vorteil, daß das erfindungsgemäße Gantry-System mit wesentlich kleinerem Radius konstruiert werden kann als ein herkömmliches System, das eine Ablenkung von 90° erfordert.

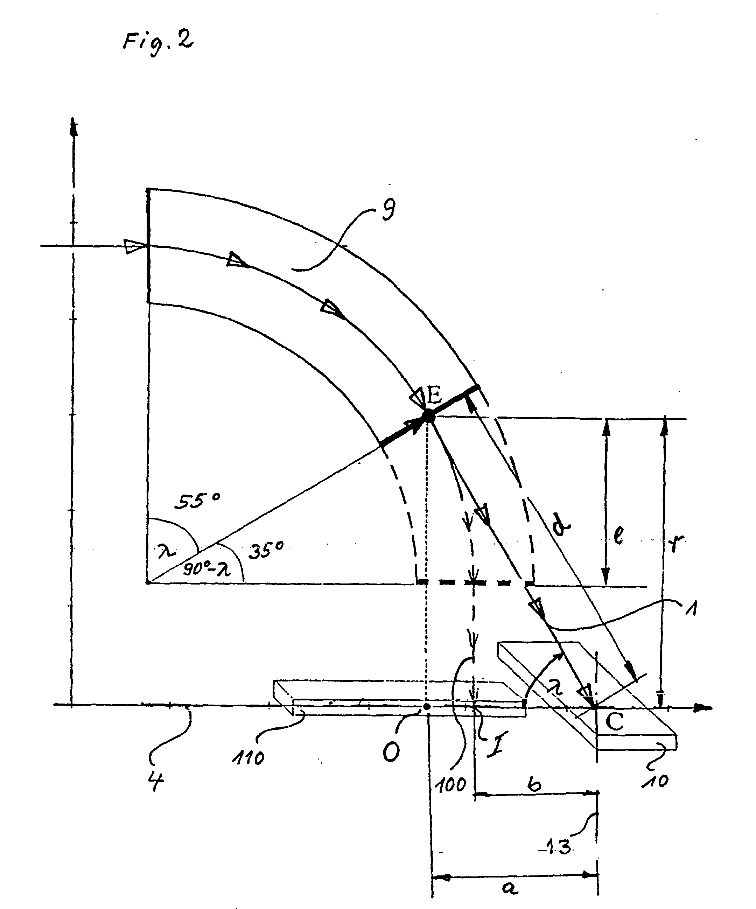

Dieser Unterschied wird deutlich durch einen Vergleich in Fig. 2 gezeigt, bei dem die Wirkung eines letzten Ablenkungsmagneten einer herkömmlichen 90°-Gantry und einer beispielsweise 55°- Gantry, die beispielswiese bei einer bevorzugten Ausführungsform des Gantry-Systems zum Einsatz kommt, gezeigt wird.

In dem Stand der Technik einer 90°-Gantry, die bisher nur für Protonen geeignet ist, wird mit dem letzten Ablenkungsmagneten der Ionenstrahl 100 aus einer Richtung parallel zur Gantryrotationsachse 4 in eine Richtung radial zur Gantryrotationsachse 4 abgelenkt und trifft je nach Drehwinkel der Gantry auf das Target unter einem einstellbaren Winkel α zwischen 0 und 360°, und durch Drehen des Targetträgers 110 kann praktisch jeder Winkel im Raum zur Bestrahlung des Targets eingestellt werden.

In diesem Ausführungsbeispiel sind bei einem Austrittswinkel aus dem letzten Ablenkungsmagneten 9 im Punkt E die Behandlungswinkel γ auf 0° bis 55° begrenzt. Dabei verschiebt sich gegenüber einer 90°-Gantry das Isozentrum 5 vom Punkt I zum Punkt C um eine beträchtliche Länge b auf der Gantry-Rotationsachse 4 aus der Gantry heraus und gleichzeitig ist der letzte Ablenkungsmagnet in seiner Größe wesentlich vermindert, so daß entweder die gesamte Gantry im Volumen verkleinert werden kann, oder der Radius, der für Targetträger 8 und für den zu behandelnden Patienten zur Verfügung steht, um die Länge 1 auf eine lichte Weite mit dem Radius r vergrößert werden kann. Tatsächlich wird der Raum, der durch Vermindern des Ablenkungswinkels des letzten Ablenkungsmagneten 9 gewonnen wird, zur Verminderung der Gesamtgröße des Gantry-Systems verwendet.

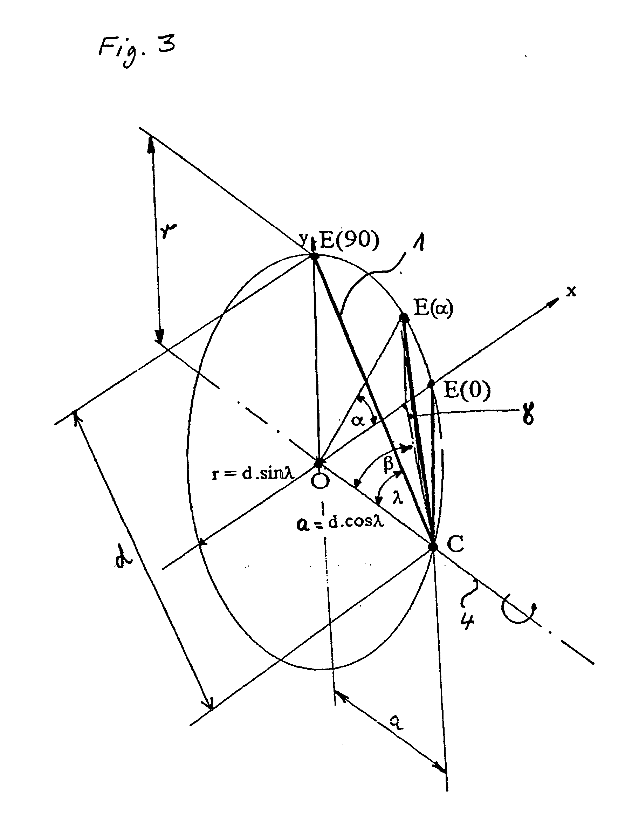

Fig. 3 zeigt dazu die Winkelverhältnisse und die Winkeldefinitionen einer Gantry, wie sie beim erfindungsgemäßen Gantry-System zum Einsatz kommen. Dazu symbolisiert die Linie 4 die Gantry-Rotationsachse, die zusammen mit der X-Achse eine horizontale Ebene aufspannt. Die Gantry-Rotationsachse wird von dem im Punkt E austretenden Ionenstrahl im Punkt C dem Isozentrum 5 geschnitten.

In Fig. 3 werden drei Positionen der Gantry und des Austrittspunktes E gezeigt, einmal, wenn das Ablenksystem für den Ionenstrahl in seiner obersten Position ist, nämlich bei E (90), dann bei einem beliebigen Winkel α bei E (α) und schließlich in einer horizontalen Ebene bei E (0). Während der Winkel α durch die Drehung der Gantry um ihre Gantry-Rotationsachse eingestellt werden kann, wird der Winkel β durch Drehen des Targetträgers vertikal zur Gantry-Rotationsachse eingestellt, wobei die Träger-Rotationsachse ebenfalls durch das Isozentrum 5 im Punkt C verläuft.

Der Winkel λ wird durch den letzten Ablenkungsmagneten vorgegeben und bleibt bei allen Drehbewegungen sowohl der Gantry-Rotationsachse als auch der Träger-Rotationsachse unverändert. Der Behandlungswinkel γ und der Eintrittskanal durch den der Strahl in den Patienten eindringt ergeben sich aus der Kombination der beiden Drehbewegungen der Gantry und des Targetträgers. Der Behandlungswinkel γ ist immer kleiner oder gleich λ und der effektive Behandlungswinkel ist entsprechend auf eine der beiden Positionen des Targetträgers begrenzt. Nimmt jedoch der Targetträger die zweite um 90° in der vertikalen Ebene verschobene Position ein, so kann der effektive Behandlungswinkel für das Target auf die in der ersten Position effektiven Behandlungswinkel eingestellt werden. Der Abstand d zwischen dem Austrittspunkt E und dem Isozentrum 5 im Punkt C bestimmt gleichzeitig in Verbindung mit dem oben beschriebenen Winkel λ die Länge a des Herausragens des Isozentrums aus der Gantry als auch die mögliche lichte Weite mit dem Radius r, die für die Gerätschaften zur Behandlung eines Patienten bei dem erfindungsgemäßen Gantry-System zur Verfügung steht.

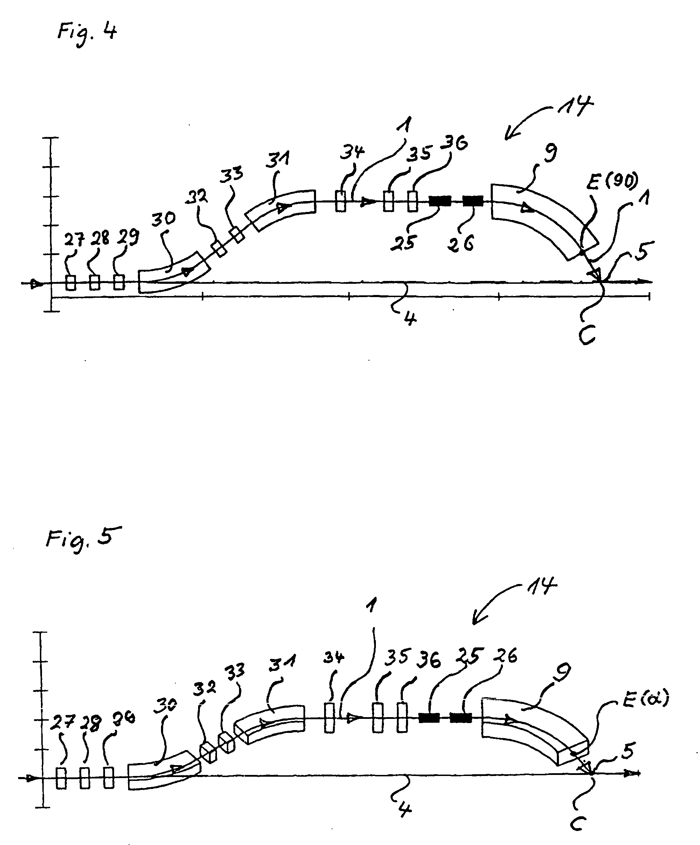

Fig. 4 zeigt die Führung des Ionenstrahls 1 der Gantry bei α = 90°, d.h. in der obersten Position der Ablenkungsmagnete. Diese Gantry wurde für einen 55°-Austrittswinkel entworfen, wobei die Ausführungsspezifikationen derart gewählt wurden, daß eine isozentrische Konstruktion ermöglicht wird und gleichzeitig ein Abtasten des Targetvolumens in zwei aktiven Richtungen, d.h. schichtweise, möglich wir, so daß diese Ausführungsform im wesentlichen folgende vorteilhafte Eigenschaften und Anordnungen aufweist:
1. achromatische Strahloptiken,
2. anpassbare Strahlgröße von 4 bis 16 mm in Schritten von 1 mm,
3. eine maximale Strahlenenergie von 400 MeV/u für einen Kohlenstroffionenstrahl mit einer Eindringtiefe in Wasser von 27 cm,
4. ein Abtastfeld von der Größe von 20 x 10 cm²,
5. ein Abtastmodus mit niedriger Winkelabweichung, d.h. der Winkel zwischen dem einfallenden Abtaststrahl und der Senkrechten zu der Abtastfläche ist kleiner als 1°,
6. eine Strahleinschnürung liegt im Isozentrum, d.h. die Strahleinschnürung im Isozentrum hat den Vorteil, daß sich der Strahlumfang nur geringfügig in der Nähe der Einschnürung ändert, so daß folglich Variätionen der Strahlbreite innerhalb eines begrenzten Tumorvolumens vernachlässigt werden können, und schließlich
7. einen minimalen Laufraum von dem Gantryaustritt zu dem Isozentrum von 1,2 m.

Eine geeignete Anordnung eines derartigen Strahltransportsystems, das die obigen Konstruktionsmerkmale aufweist, wird in Fig. 4 gezeigt. Diese Gantry 14 besteht aus einem geraden Anpassungsabschnitt, in dem der Ionenstrahl in die Gantry-Rotationsachse 4 eingeführt wird. Dieser Eingangs-Anpassungsabschnitt besteht aus drei Quadrupolen 27 bis 29. Weiterhin gibt es eine Biegeschulter aus zwei 38°-Ablenkungsmagneten 30 und 31 mit dazwischenliegenden zwei Quadrupolen 32, 33, einen oberen geraden Abschnitt, in dem der Ionenstrahl parallel zur Gantry-Rotationsachse 4 geführt wird, mit drei weiteren Quadrupolen 34 bis 36 und zwei Abtastmagneten mit horizontaler 25 und vertikaler 26 Ablenkung für ein Abtasten des Targetvolumens und einen letzten 55°-Ablenkungsmagnet 9 mit großer Apertur.

Der Freiraum für den Patienten hat einen Radius von ungefähr 1,2 m, der Außenradius der Gantry beträgt etwa 2,8 m. Im wesentlichen wird vorteilhaft die Strahlgröße hauptsächlich durch die ersten drei Quadrupole, die in dem beugungsfreien Bereich des Eingangsabschnitts angeordnet sind, bestimmt. Eine derartige Anordnung zeigt einen hohen Grad der ionenoptischen Flexibilität. Der Strahl im Isozentrum kann von 2 bis 16 mm im Durchmesser eingeschnürt werden und die Optiken der Gantry arbeiten immer in einem achromatischen Zustand.

Fig. 5 zeigt den gleichen Aufbau mit den gleichen Komponenten, wie Fig. 4 für eine Führung des Ionenstrahls bei einem Winkel α von 45°. Dazu sind in Fig. 5 gleiche Elemente mit gleichen Bezugszeichen gekennzeichnet.

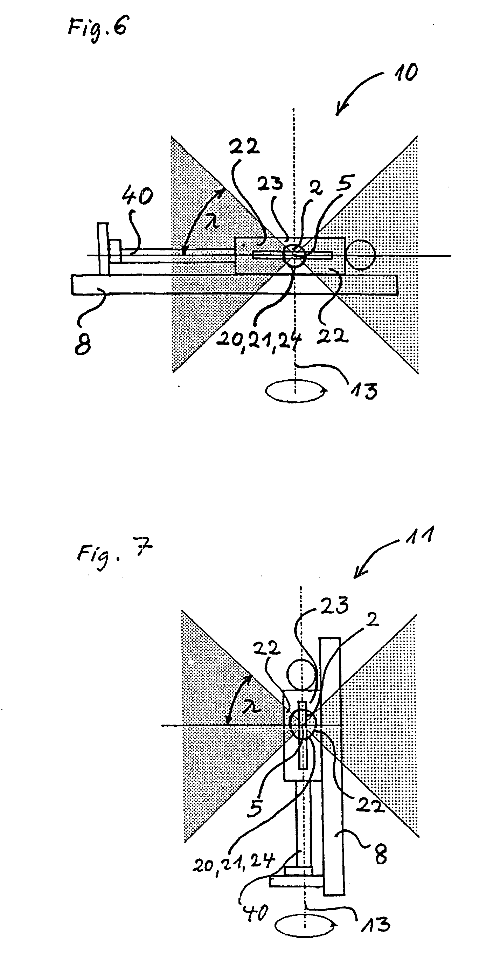

Fig. 6 zeigt eine Seitenansicht von einer der beiden möglichen Positionen des Targetträgers 8. Der Targetträger 8 ist in Fig. 6 in einer horizontalen Ebene angeordnet und kann um eine vertikale Achse 13 gedreht werden. Der Targetträger trägt einen Patienten 40 mit einem Tumorvolumen 20. Der Patient wird auf dem horizontalen Targetträger einer Patientencouch oder einem Patiententisch derart angeordnet, daß die vertikale Träger-Rotationsachse 13 durch das Tumorvolumen 20 geht, und der Targetträger 8 wird mit Hilfe des Targetträger-Systems in das Isozentrum 5 des Gantry-Systems verbracht, so daß das Tumorvolumen 20 im Isozentrum 5 liegt.

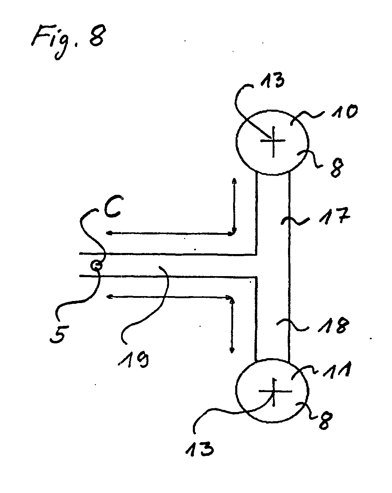

Fig. 6 zeigt als punktierte Flächen die Bereiche, die mit der Position 10 des Targetträgers als effektive Behandlungswinkel in einer bevorzugten Ausführungsform der Erfindung erreichbar sind, während Fig. 7 die zweite Position des Targetträgers 8 zeigt und die weiteren Bereiche durch punktierte Flächen eines möglichen effektiven Behandlungswinkels einer Ausführungsform des erfindungsgemäßen Gantry-Systems darstellt.

In den Figuren 6 und 7 wird deutlich zwischen dem zu durchstrahlenden gesunden Gewebe 22, sowie dem nicht betroffenen umgebenden Gewebe 23 und dem zu bestrahlenden Tumorgewebe 24 schematisch unterschieden. Die punktierten Bereiche der Figuren 6 und 7 sind für einen Ablenkungswinkel von 45° gezeichnet und verdeutlichen, daß auch bei einem derart geringen Ablenkungswinkel von 45° des letzten Ablenkungsmagneten ein Ionenstrahl auf ein Target aus beliebig bestimmbarem effektiven Behandlungswinkel eingestellt und ausgerichtet werden kann.

Fig. 8 zeigt eine Anordnung von Zuführschienen 17, 18 und 19 gemäß einer weiteren Ausführungsform der Erfindung. Auf den beiden Zuführschienen 17 und 18 sind einander gegenüber jeweils ein Targetträger 8 in einer ersten Position 10, in der der Targetträger horizontal ausgerichtet ist, und in einer weiteren Position 11, in der der Targetträger senkrecht ausgerichtet ist, angeordnet. Die Targetträger sind jeweils um ihre vertikalen Achsen 13 drehbar.

Ein Patient kann, nachdem der günstigste effektive Behandlungswinkel zur Bestrahlung eines Tumorvolumens bestimmt ist; in einer notwendigen Position 10 oder 11 des Targetträgers 8 angeordnet werden. Der Targetträger 8 mit der richtigen Position 10 oder 11 wird dann auf einer der beiden Zuführschienen 17 oder 18 zunächst bis zu einer zentralen Zuführschiene 19 verfahren und dann in Richtung auf das Isozentrum 5 des Gantry-Systems entlang der Zuführschiene 19 verschoben, so daß die Träger-Rotationsachse des geeigneten Targetträgers in dem Isozentrum des Gantry-Systems positioniert ist.

Das Ausrichten und Einstellen des Targetträgers 8 durch Drehen des Targetträgers 8 um seine Träger-Rotationsachse 13 in bezug auf den günstigsten Eintrittskanal kann vor oder nach dem Verschieben in das Isozentrum 5 erfolgen. Auch das Ausrichten und Einstellen der Gantry durch Drehen der Gantry um ihre horizontale Gantry-Rotationsachse in bezug auf den günstigsten Behandlungswinkel kann ebenfalls vor oder nach dem Verschieben des Patienten auf dem Targetträger in das Isozentrum erfolgen. Erst wenn die Einstellung des geeigneten Behandlungswinkels und Eintrittkanals durch Auswahl der richtigen Position eines Targetträgers 8 und durch Drehen des Targetträgers um seine Träger-Rotationsachse 13 als auch durch Drehen der Gantry um ihre horizontale Gantry-Rotationsachse abgeschlossen ist, kann ein räumliches Abtasten des gesamten Tumorvolumens mit dem Ionenstrahl erfolgen.

## Patentansprüche

1. Gantry-System zum Einstellen und Ausrichten eines Ionenstrahls (1) auf ein Target (2) aus einem beliebig bestimmbaren effektiven Behandlungswinkel, wobei der Ionenstrahl (1) in der horizontal angeordneten Gantry-Rotationsachse (4) dem Gantry-System (6) zugeführt und mittels Magnetoptiken von der Gantry-Rotationsachse (4) zunächst abgelenkt wird und dann unter einstellbaren Winkeln von 0 bis 360° um die Gantry-Rotationsachse (4) auf ein Target (2) so ausrichtbar ist, daß der Ionenstrahl (1) die Gantry-Rotationsachse (4) in dem Isozentrum (5) des Gantry-Systems (6) schneidet, wobei das Gantry-System (6) eine Gantry (14) und ein Targetträger-System (7) mit einem drehbaren Targetträger (8), dessen Träger-Rotationsachse (13) vertikal zu der Gantry-Rotationsachse (4) in dem Isozentrum angeordnet ist, aufweist
**dadurch gekennzeichnet,**
**daß** der letzte Ablenkungsmagnet (9) den Ionenstrahl (1) derart ablenkt, daß er unter einem Winkel zwischen größer gleich 45° und kleiner 90° die Gantry-Rotationsachse (4) im Isozentrum (5) schneidet, so daß der Ionenstrahl (1) beim Drehen der Gantry (14) um eine volle Umdrehung um die Gantry-Rotationsachse (4) einen Kegelmantel beschreibt und das Targetträger-System (7) zwei Positionen (10, 11), die in vertikaler Ebene senkrecht zueinander sind, für einen Targetträger (8) aufweist, wobei dessen Träger-Rotationsachse (13) in das Isozentrum (5) des Gantry-Systems (6) bringbar ist.

2. Gantry-System nach Anspruch 1, **dadurch gekennzeichnet, daß** der letzte Ablenkungsmagnet (9) den Ionenstrahl (1) derart ablenkt, daß der Ionenstrahl (1) die Gantry-Rotationsachse (4) unter einem Winkel größer gleich 45° und kleiner 90° schneidet.

3. Gantry-System nach Anspruch 1 oder 2, **dadurch gekennzeichnet, daß** das Targetträger-System (7) eine Drehbühne (15), die um eine vertikale Drehbühnenachse (16) drehbar ist, aufweist, auf der zwei Targetträger (8) in zwei Positionen (10, 11), die in vertikaler Ebene senkrecht zueinander sind, angeordnet sind und die jeweils um eine vertikale Träger-Rotationsachse (13) drehbar sind.

4. Gantry-System nach einem der Ansprüche 1 bis 3, **dadurch gekennzeichnet, daß** die vertikalen Träger-Rotationsachsen (13) der zwei Positionen (10, 11) der Targetträger (8) wechselweise in das Isozentrum (5) des Gantry Systems (6) mittels einer Drehbühne (15) durch Drehen um eine Drehbühnenachse (16) bringbar sind.

5. Gantry-System nach Anspruch 1 oder 2, **dadurch gekennzeichnet, daß** das Targetträger-System (7) zwei getrennte Zuführschienen (17, 18) aufweist, auf denen jeweils ein Targetträger (8) der zwei Positionen (10, 11) angeordnet ist, wobei die Zuführschienen (17, 18) aus jeweils unterschiedlichen Richtungen jeweils einen der Targetträger (8) mit seiner Träger-Rotationsachse (13) in das Isozentrum (5) wechselweise lateral verschieben können.

6. Gantry-System nach Anspruch 1 oder 2, **dadurch gekennzeichnet, daß** das Träger-System (7) ein universales Roboter-System (19) aufweist, das den Targetträger (8) in den zwei Positionen (10, 11) und die vertikalen Träger-Rotationsachsen (13) in dem Isozentrum (5) anordnet.

## Claims

1. Gantry system for adjusting and aligning an ion beam (1) onto a target (2) from a freely determinable effective treatment angle, wherein the ion beam (1) is supplied to the gantry system (6) in the horizontally arranged gantry rotation axis (4) and firstly is deflected away from the gantry rotation axis (4) by means of magnetic optics and then is arranged to be so aligned onto a target (2) at adjustable angles of from 0 to 360° around the gantry rotation axis (4) that the ion beam (1) intersects the gantry rotation axis (4) in the isocentre (5) of the gantry system (6), the gantry system (6) having a gantry (14) and a target carrier system (7) which has a rotatable target carrier (8), the carrier rotation axis (13) of which is arranged in the isocentre in a vertical direction with respect to the gantry rotation axis (4),
**characterised in that**
the final deflection magnet (9) so deflects the ion beam (1) that it intersects the gantry rotation axis (4) in the isocentre (5) at an angle of between greater than or equal to 45° and less than 90°, so that the ion beam (1) describes a surface of a cone on rotation of the gantry (14) through a full revolution about the gantry rotation axis (4), and the target carrier system (7) has two positions (10, 11) for a target carrier (8), which positions are perpendicular to one another in a vertical plane, the carrier rotation axis (13) of which target carrier (8) can be brought into the isocentre (5) of the gantry system (6).

2. Gantry system according to claim 1, **characterised in that** the final deflection magnet (9) so deflects the ion beam (1) that the ion beam (1) intersects the gantry rotation axis (4) at an angle greater than or equal to 45° and less than 90°.

3. Gantry system according to claim 1 or 2, **characterised in that** the target carrier system (7) has a revolving platform (15), which is rotatable about a vertical revolving platform axis (16), on which two target carriers (8) are arranged in two positions (10, 11), which are perpendicular to one another in a vertical plane, and each of which is rotatable about a vertical carrier rotation axis (13).

4. Gantry system according to one of claims 1 to 3, **characterised in that** the vertical carrier rotation axes (13) of the two positions (10,11) of the target carriers (8) are arranged to be brought alternately into the isocentre (5) of the gantry system (6) by means of a revolving platform (15) as a result of rotation about a revolving platform axis (16).

5. Gantry system according to claim 1 or 2, **characterised in that** the target carrier system (7) has two separate delivery rails (17, 18), on each of which there is arranged one target carrier (8) of the two positions (10,11), the delivery rails (17, 18) being capable of laterally displacing, from different directions in each case, either of the target carriers (8) with its carrier rotation axis (13) into the isocentre (5) alternately.

6. Gantry system according to claim 1 or 2, **characterised in that** the carrier system (7) has a universal robot system (19), which arranges the target carrier (8) in the two positions (10, 11) and arranges the vertical carrier rotation axes (13) in the isocentre (5).

## Revendications

1. Système à portique pour régler et orienter un faisceau d'ions (1) sur une cible (2) à partir d'un angle effectif de traitement pouvant être fixé à volonté, sachant que le faisceau d'ions (1) est amené dans l'axe de rotation, disposé horizontalement, du portique (4) du système à portique (6) et est d'abord dévié au moyen d'optiques magnétiques de l'axe de rotation du portique (4), puis peut être dirigé sur une cible (2) sous des angles réglables de 0 à 360° autour de l'axe de rotation du portique (4) de telle sorte que le faisceau d'ions (1) coupe l'axe de rotation du portique (4) dans l'isocentre (5) du système à portique (6), sachant que le système à portique (6) comprend un portique (14) et un système de support de cible (7) avec un support de cible (8) tournant dont l'axe de rotation du support (13) est disposé verticalement dans l'isocentre par rapport à l'axe de rotation du portique (4),
**caractérisé en ce que**
le dernier aimant de déviation (9) dévie le faisceau d'ions (1) de telle sorte qu'il coupe l'axe de rotation du portique (4) dans l'isocentre (5) sous un angle compris entre supérieur ou égal à 45° et inférieur à 90°, de telle sorte que, lors de la rotation du portique (14) d'un tour complet autour de l'axe de rotation du portique (4), le faisceau d'ions (1) décrit la surface périphérique d'un cône et que le système de support de cible (7) présente pour un support de cible (8) deux positions (10, 11) qui sont perpendiculaires l'une à l'autre dans le plan vertical, sachant que son axe de rotation du support (13) peut être amené dans l'isocentre (5) du système à portique (6).

2. Système à portique selon la revendication 1, **caractérisé en ce que** le dernier aimant de déviation (9) dévie le faisceau d'ions (1) de telle sorte que le faisceau d'ions (1) coupe l'axe de rotation du portique (4) sous un angle supérieur ou égal à 45° et inférieur à 90°.

3. Système à portique selon la revendication 1 ou 2, **caractérisé en ce que** le système de support de cible (7) comporte un plateau tournant (15), qui peut tourner autour d'un axe vertical de plateau tournant (16), sur lequel deux supports de cible (8) sont disposés dans deux positions (10, 11) qui sont perpendiculaires l'une par rapport à l'autre dans le plan vertical, et qui peuvent tourner chacune autour d'un axe vertical de rotation de support (13).

4. Système à portique selon l'une quelconque des revendications 1 à 3, **caractérisé en ce que** les axes de rotation verticaux des supports (13) des deux positions (10, 11) des supports de cible (8) peuvent être amenés alternativement dans l'isocentre (5) du système à portique (6) au moyen d'un plateau tournant (15) par rotation autour d'un axe de plateau tournant (16).

5. Système à portique selon la revendication 1 ou 2, **caractérisé en ce que** le système de support de cible (7) comporte deux glissières d'alimentation (17, 18) séparées, sur chacune desquelles est disposé un support de cible (8) des deux positions (10, 11), sachant que chacune des glissières d'alimentation (17, 18) peut alternativement translater latéralement depuis des directions respectivement différentes un des supports de cible (8) avec son axe de rotation de support (13) dans l'isocentre (5).

6. Système à portique selon la revendication 1 ou 2, **caractérisé en ce que** le système de support (7) comporte un système universel (19) à robot qui dispose le support de cible (8) dans les deux positions (10, 11) et les axes de rotation verticaux des supports (13) dans l'isocentre (5).
